# EUROPEAN PATENT SPECIFICATION

(11) **EP 3 811 844 B1**
(45) Date of publication and mention of the grant of the patent: **13.08.2025**
(21) Application number: 20199087.6
(22) Date of filing: 29.09.2020
(51) Int. Cl.: A61B 1/12, A61B 18/14, A61B 90/70, A61B 90/96, A61B 90/98, A61B 18/00, A61B 1/00

(54) **LIQUID SUPPLY DEVICE FOR ENDOSCOPE, LIQUID SUPPLY TUBE, AND LIQUID SUPPLY DEVICE BODY**
FLÜSSIGKEITSVERSORGUNGSVORRICHTUNG FÜR ENDOSKOP, FLÜSSIGKEITSVERSORGUNGSSCHLAUCH UND FLÜSSIGKEITSVERSORGUNGSVORRICHTUNGSKÖRPER
DISPOSITIF D'ALIMENTATION EN LIQUIDE POUR ENDOSCOPE, TUBE D'ALIMENTATION EN LIQUIDE ET CORPS DE DISPOSITIF D'ALIMENTATION EN LIQUIDE

(30) Priority: 30.09.2019 JP 2019179132
(43) Date of publication of application: 28.04.2021
(73) Proprietor: FUJIFILM Corporation, Tokyo 106-8620 (JP)
(72) Inventor: FUJIHIRA, Takuro, Japan, Kanagawa 258-8538 (JP); SATO, Jun, Japan, Kanagawa 258-8538 (JP)
(74) Representative: Dehns Germany Partnerschaft mbB

(56) References cited:
- US-A- 5 460 490
- US-A1- 2011 196 291
- US-A1- 2013 008 233
- US-A1- 2013 131 579
- US-A1- 2015 216 608

## Description

### BACKGROUND OF THE INVENTION

### 1. Field of the Invention

The present invention relates to a liquid supply device for an endoscope, a liquid supply tube, and a liquid supply device body, and more particularly, to a liquid supply device for an endoscope, a liquid supply tube, and a liquid supply device body that are used to send liquid to a liquid supply pipe line provided in an endoscope or a treatment tool.

### 2. Description of the Related Art

In the field of an endoscope in the related art, liquid is supplied into a body to be observed using a liquid supply pipe line, such as a treatment tool-insertion channel or a water jet pipe inserted into an endoscope. For example, JP2006-325814A discloses a liquid supply device for an endoscope that sends liquid to the liquid supply pipe line. The liquid supply device for an endoscope sends liquid to the liquid supply pipe line by rotating a rotor and squeezing a part of the liquid supply tube with rollers arranged on the outer peripheral portion of the rotor in a state where the liquid supply tube is wound on the outer peripheral portion of the rotor.

Further, the liquid supply device for an endoscope disclosed in JP2006-325814A comprises a flow rate adjustment knob, and the amount of liquid to be supplied to the liquid supply pipe line can be adjusted by the operation of the flow rate adjustment knob. US 2015/0216608 discloses an adapter identification of a reprocessing device for surgical instruments. US 5460490 discloses a multi-purpose irrigation/aspiration pump system. US 2011/196291 discloses an apparatus and method for recognizing coupling between two system components. US 2013/0131579 discloses a system for identifying the presence and correctness of a medical device accessory. US 2013/008233 discloses a water leakage checking apparatus.

### SUMMARY OF THE INVENTION

Meanwhile, liquid supply pipe lines (liquid supply pipe lines, such as treatment tool-insertion channels or water jet pipes) corresponding to a liquid supply destination to which liquid is to be sent from the liquid supply device for an endoscope generally have inner diameters that are different from each other due to the purpose or the intended use, design restrictions, or the like.

Further, for example, the liquid supply pipe line of a treatment tool, such as a high-frequency knife having a water supply function, may also be a liquid supply destination to which liquid is to be supplied from the liquid supply device for an endoscope. For this reason, it is desirable that a plurality of types of liquid supply tubes having, for example, different inner diameters or the like can be connected so as to be interchangeable and can be used in the liquid supply device for an endoscope according to the type of a liquid supply pipe line corresponding to the liquid supply destination.

However, in a case where a desired liquid supply tube among a plurality of types of liquid supply tubes is connected and used in the liquid supply device for an endoscope disclosed in JP2006-325814A, a manipulator, such as an operator, should operate the flow rate adjustment knob and adjust the amount of liquid to be supplied in order to set the amount of liquid to be supplied suitable for the type of a liquid supply pipe line corresponding to the liquid supply destination and this operation is cumbersome. For this reason, there is a problem that the convenience for a manipulator is insufficient.

The invention has been made in consideration with the above-mentioned circumstances, and an object of the invention is to provide a liquid supply device for an endoscope, a liquid supply tube, and a liquid supply device body of which convenience can be improved.

According to an aspect of the present invention, there is provided a liquid supply tube as claimed in claim 1.

In the liquid supply device for an endoscope according to this aspect of the invention, it is preferable that the flow rate-setting unit sets the flow rate of the liquid in a flow rate range different for each type of the liquid supply tube, and the liquid supply device comprises an adjustment unit allowing the flow rate of the liquid to be manually adjusted in the flow rate range set by the flow rate-setting unit.

In the liquid supply device for an endoscope according to this aspect of the invention, it is preferable that, in a state where the liquid supply tube is wound on a rotor including a plurality of rollers provided on an outer peripheral portion thereof, the pump sends liquid by rotating the rotor and squeezing the liquid supply tube with the rollers.

In the liquid supply device for an endoscope according to this aspect of the invention, it is preferable that the identifier has mechanical identification information different for each type of the liquid supply tube and the identifier recognition unit is a mechanical reading unit capable of mechanically reading the mechanical identification information.

**In** the liquid supply device for an endoscope according to this aspect of the invention, it is preferable that the identifier has magnetic identification information different for each type of the liquid supply tube and the identifier recognition unit is a magnetic reading unit capable of magnetically reading the magnetic identification information.

**In** the liquid supply device for an endoscope according to this aspect of the invention, it is preferable that the identifier has electric identification information different for each type of the liquid supply tube and the identifier recognition unit is an electric reading unit capable of electrically reading the electric identification information.

**In** the liquid supply device for an endoscope according to this aspect of the invention, it is preferable that the identifier has optical identification information different for each type of the liquid supply tube and the identifier recognition unit is an optical reading unit capable of optically reading the optical identification information.

In the liquid supply device for an endoscope according to this aspect of the invention, it is preferable that the liquid supply tube includes at least one joint of a first joint to be connected to a first tube corresponding to a liquid destination side or a second joint to be connected to a second tube corresponding to a liquid source side and the identifier is provided on the one joint.

In the liquid supply device for an endoscope according to this aspect of the invention, it is preferable that the part to which liquid is to be supplied is a liquid supply pipe line provided in an endoscope or a treatment tool.

In order to achieve the object of the invention, a liquid supply tube according to another aspect of the invention is used for a liquid supply device for an endoscope including a liquid supply device body, to which a plurality of types of liquid supply tubes are connected so as to be interchangeable, and sends liquid to a part to which liquid is to be supplied by a pump provided in the liquid supply device body. The liquid supply tube comprises an identifier that is used to identify the type of the liquid supply tube, and the identifier is provided at a position that is capable of being recognized by an identifier recognition unit provided in the liquid supply device body in a case where the liquid supply tube is connected to the liquid supply device body.

In the liquid supply tube according to this aspect of the invention, it is preferable that the identifier has mechanical identification information different for each type of the liquid supply tube and the identifier recognition unit is a mechanical reading unit capable of mechanically reading the mechanical identification information.

In the liquid supply tube according to this aspect of the invention, it is preferable that the identifier has magnetic identification information different for each type of the liquid supply tube and the identifier recognition unit is a magnetic reading unit capable of magnetically reading the magnetic identification information.

In the liquid supply tube according to this aspect of the invention, it is preferable that the identifier has electric identification information different for each type of the liquid supply tube and the identifier recognition unit is an electric reading unit capable of electrically reading the electric identification information.

In the liquid supply tube according to this aspect of the invention, it is preferable that the identifier has optical identification information different for each type of the liquid supply tube and the identifier recognition unit is an optical reading unit capable of optically reading the optical identification information.

In the liquid supply tube according to this aspect of the invention, it is preferable that the part to which liquid is to be supplied is a liquid supply pipe line provided in an endoscope or a treatment tool.

In order to achieve the object of the invention, a liquid supply device body according to still another aspect of the invention is a liquid supply device body to which a plurality of types of liquid supply tubes are connected so as to be interchangeable and in which the liquid supply tube is provided with an identifier used to identify the type of the liquid supply tube. The liquid supply device body comprises a pump that sends liquid to a part to which liquid is to be supplied through the liquid supply tube, an identifier recognition unit that recognizes the identifier, and a flow rate-setting unit that sets a flow rate of the liquid to be supplied to the part to which liquid is to be supplied for each type of the liquid supply tube by changing an operating condition of the pump on the basis of the identifier recognized by the identifier recognition unit.

In the liquid supply device body according to this aspect of the invention, it is preferable that the flow rate-setting unit sets the flow rate of the liquid in a flow rate range different for each type of the liquid supply tube and the liquid supply device comprises an adjustment unit allowing the flow rate of the liquid to be manually adjusted in the flow rate range set by the flow rate-setting unit.

In the liquid supply device body according to this aspect of the invention, it is preferable that, in a state where the liquid supply tube is wound on a rotor including a plurality of rollers provided on an outer peripheral portion thereof, the pump sends liquid by rotating the rotor and squeezing the liquid supply tube with the rollers.

In the liquid supply device body according to this aspect of the invention, it is preferable that the identifier has mechanical identification information different for each type of the liquid supply tube and the identifier recognition unit is a mechanical reading unit capable of mechanically reading the mechanical identification information.

In the liquid supply device body according to this aspect of the invention, it is preferable that the identifier has magnetic identification information different for each type of the liquid supply tube and the identifier recognition unit is a magnetic reading unit capable of magnetically reading the magnetic identification information.

In the liquid supply device body according to this aspect of the invention, it is preferable that the identifier has electric identification information different for each type of the liquid supply tube and the identifier recognition unit is an electric reading unit capable of electrically reading the electric identification information.

In the liquid supply device body according to this aspect of the invention, it is preferable that the identifier has optical identification information different for each type of the liquid supply tube and the identifier recognition unit is an optical reading unit capable of optically reading the optical identification information.

In the liquid supply device body according to this aspect of the invention, it is preferable that the part to which liquid is to be supplied is a liquid supply pipe line provided in an endoscope or a treatment tool.

According to the invention, convenience can be improved.

### BRIEF DESCRIPTION OF THE DRAWINGS

Fig. 1 is a diagram showing the entire configuration in which a liquid supply device for an endoscope according to an embodiment is shown together with an endoscope.
Fig. 2 is a diagram showing tube units that are connected to the endoscope and a treatment tool.
Fig. 3 is a diagram showing the configuration of an endoscope system comprising the endoscope.
Fig. 4 is a perspective view of the entire liquid supply device body.
Fig. 5 is an enlarged view of main portions of the liquid supply device body of which a pump head cover is open.
Fig. 6 is a perspective view showing the configuration of the tube unit.
Fig. 7 is a perspective view of which main portions are enlarged and which shows the shapes of restriction portions.
(A), (B), and (C) of Fig. 8 are diagrams showing the configuration of identifiers that are used to identify the types of liquid supply tubes.
Fig. 9 is a diagram showing that a liquid supply tube is attached to the liquid supply device body.
Fig. 10 is a control block diagram relating to the control of the amount of liquid to be supplied by the liquid supply device body.
Fig. 11 is a diagram showing examples of another identification information and another reading unit.

### DESCRIPTION OF THE PREFERRED EMBODIMENTS

A liquid supply device for an endoscope, a liquid supply tube, and a liquid supply device body according to embodiments of the invention will be described below with reference to the accompanying drawings.

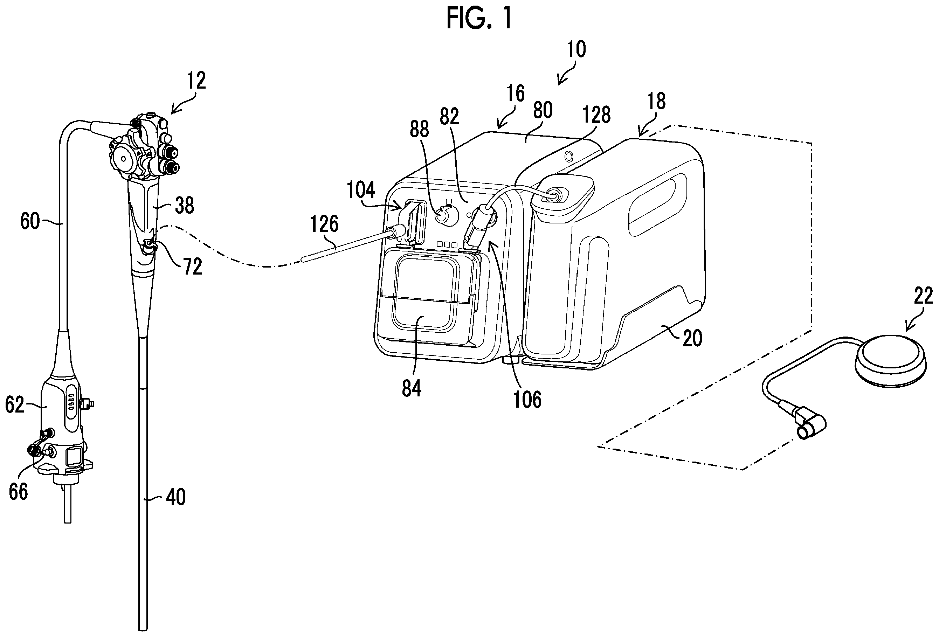

Fig. 1 is a diagram showing the entire configuration in which a liquid supply device 10 for an endoscope according to an embodiment is shown together with an endoscope 12.

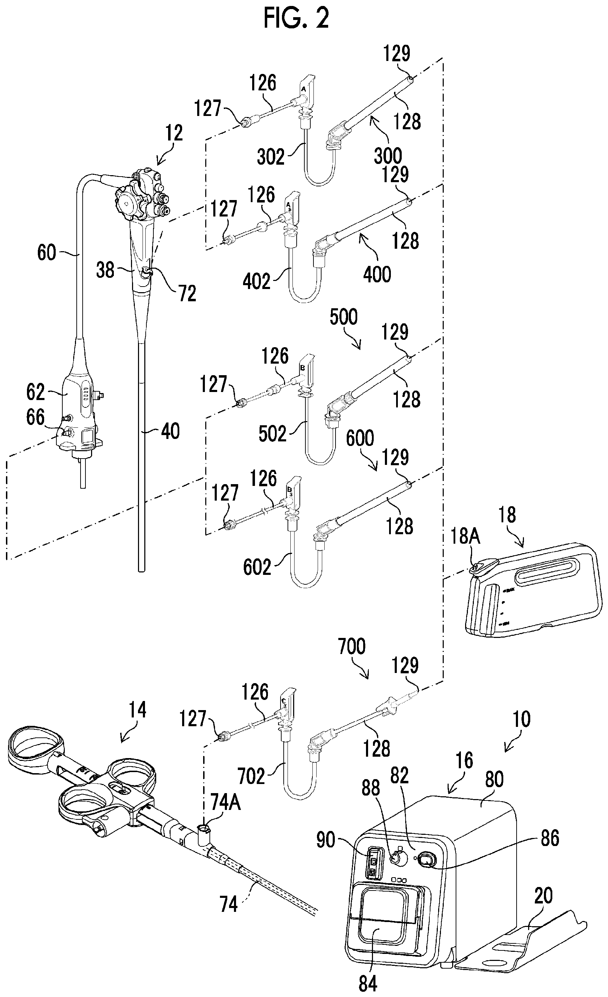

Fig. 2 is a diagram showing four different types of tube units 300, 400, 500, and 600 that are connected to a liquid supply pipe line of the endoscope 12 and a tube unit 700 that is connected to a liquid supply pipe line of a high-frequency knife 14 that is a treatment tool. These tube units 300 to 700 are connected to a liquid supply device body 16 forming the liquid supply device 10 for an endoscope so as to be interchangeable depending on the purpose or the intended use.

Further, the liquid supply device 10 for an endoscope includes a tank 18 in which liquid, such as a normal saline solution or a chemical solution, is stored, and the tank 18 is used in a state where the tank 18 is attachably and detachably placed on a tank tray 20 provided on the liquid supply device body 16. The liquid supply device body 16 and the tube units 300 to 700 will be described later.

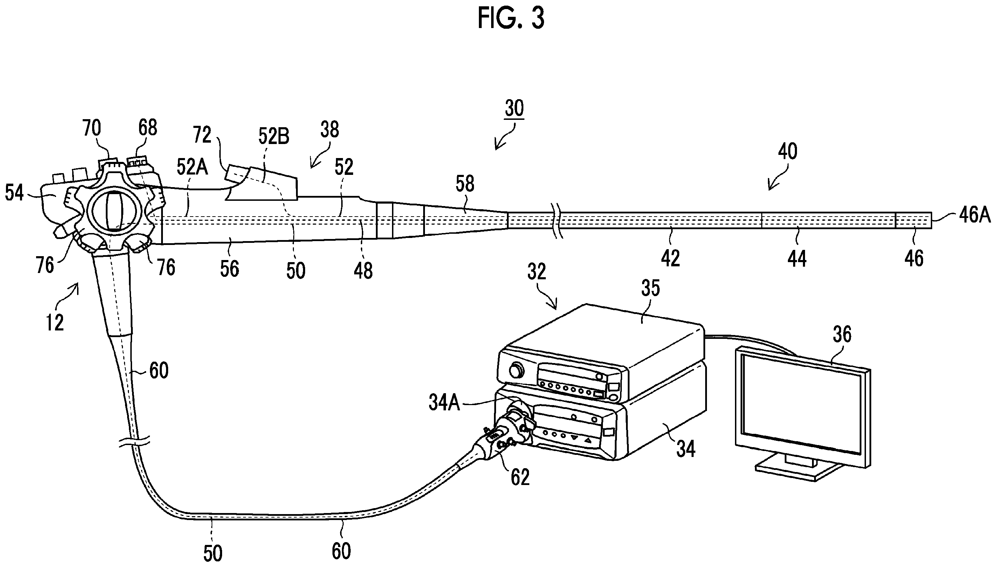

Next, an example of the endoscope 12 to be connected to the liquid supply device 10 for an endoscope will be described with reference to Fig. 3.

Fig. 3 is a diagram showing the configuration of an endoscope system 30 comprising the endoscope 12. The endoscope system 30 comprises the endoscope 12, a processor device 32 for an endoscope, and a display 36.

The endoscope 12 comprises an operation unit 38 and an insertion unit 40 that is provided on the distal end side of the operation unit 38 and is to be inserted into a body to be observed.

The insertion unit 40 includes a soft part 42, a bendable part 44, and a distal end part 46 that are arranged in this order toward a distal end from a proximal end. An air/water supply channel 48, a water jet pipe 50, and a treatment tool-insertion channel 52 shown in Fig. 3 by a dotted line, and the like are provided in the insertion unit 40. The air/water supply channel 48 is formed of a pipe line that jets liquid or air to an observation window (not shown) provided on a distal end face 46A of the distal end part 46 through a nozzle (not shown). The water jet pipe 50 is formed of a liquid supply pipe line that supplies liquid into the body to be observed, and the treatment tool-insertion channel 52 includes a pipe line into which a treatment tool is to be inserted and a liquid supply pipe line that supplies liquid into the body to be observed.

The operation unit 38 includes an operation unit body 54 and a grip part 56 connected to the operation unit body 54, and the proximal end portion of the insertion unit 40 is connected to the distal end side of the grip part 56 through a bending-proof pipe 58. The grip part 56 is a part that is gripped by an operator during an examination using the endoscope 12.

The operation unit body 54 is provided with a universal cable 60. A connector unit 62 is provided on the distal end side of the universal cable 60, and is connected to the processor device 32 for an endoscope. The processor device 32 for an endoscope comprises a light source device 34 and an image processing device 35. The light source device 34 is provided with a processor-side connector 34A to which the connector unit 62 is to be connected. Further, a display 36, which displays an image subjected to image processing by the image processing device 35, is connected to the image processing device 35. The endoscope system 30 is adapted to transmit electric power, optical signals, and the like between the endoscope 12 and the processor device 32 for an endoscope through a connector part, which includes the connector unit 62 and the processor-side connector 34A, in a non-contact manner. Accordingly, light generated from the light source device 34 is transmitted through an optical fiber cable (not shown), and is emitted from an illumination window (not shown) that is provided on the distal end face 46A of the distal end part 46. Furthermore, the optical signals of an image taken from the observation window are subjected to image processing by the image processing device 35 and are displayed on the display 36 as an image.

Here, as shown in Fig. 2, the connector unit 62 is provided with a liquid supply connector 66. A proximal end opening of the water jet pipe 50 (see Fig. 3) inserted into the insertion unit 40 and the universal cable 60 is connected to the liquid supply connector 66. A distal end opening of the water jet pipe 50 is disposed on the distal end face 46A of the distal end part 46 as a jet port. Two types of the tube unit 500 and the tube unit 600 are connected to the liquid supply connector 66 shown in Fig. 2 so as to be interchangeable.

Returning to Fig. 3, an air/water supply button 68 and a suction button 70 are installed side by side on the operation unit body 54. In a case where the air/water supply button 68 is operated by an operator, liquid or air is supplied to the air/water supply channel 48. Accordingly, liquid or air is jetted to the observation window. Further, in a case where the suction button 70 is operated by an operator, liquid present in the body to be observed is sucked from a distal end opening of the treatment tool-insertion channel 52, which is disposed on the distal end face 46A of the distal end part 46, through the treatment tool-insertion channel 52.

The treatment tool-insertion channel 52 branches into a suction passage 52A and a treatment tool-insertion passage 52B in the grip part 56. The suction passage 52A is connected to a suction port (not shown) of the connector unit 62 through a cylinder (not shown) of the suction button 70 and the universal cable 60, and a suction pump (not shown) is connected to the suction port. Furthermore, the treatment tool-insertion passage 52B is connected to a forceps port 72 protruding from the grip part 56. Two types of the tube unit 300 and the tube unit 400 shown in Fig. 2 are connected to the forceps port 72 so as to be interchangeable.

Further, the high-frequency knife 14 shown in Fig. 2 is introduced into the treatment tool-insertion channel 52 from the forceps port 72 shown in Fig. 3 through the treatment tool-insertion passage 52B. The high-frequency knife 14 is provided with a liquid supply pipe line 74 shown in Fig. 2 by a dotted line, and the tube unit 700 is connected to a proximal end opening 74A of the liquid supply pipe line 74.

Returning to Fig. 3, the operation unit body 54 is provided with a pair of angle knobs 76 and 76 that is used to perform an operation for bending the bendable part 44. The pair of angle knobs 76 and 76 is coaxially provided so as to be rotationally movable.

The schematic configuration of the endoscope system 30 shown in Fig. 3 has been described above. The liquid supply device 10 for an endoscope shown in Figs. 1 and 2 will be described below.

As shown in Fig. 1, the liquid supply device 10 for an endoscope comprises the liquid supply device body 16, the tank 18, and a foot switch 22. Five different types of tube units 300 to 700 shown in Fig. 2 are connected to the liquid supply device body 16 so as to be interchangeable depending on the purpose or the intended use.

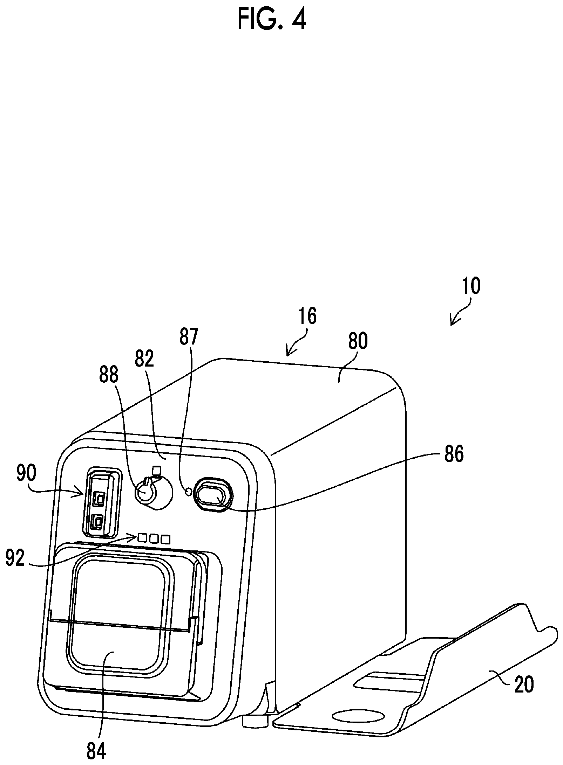

Fig. 4 is a perspective view of the entire liquid supply device body 16.

As shown in Fig. 4, the liquid supply device body 16 includes a housing 80. A pump head cover (hereinafter abbreviated as a "cover") 84, a power switch 86, a power switch indicator lamp 87, a flow rate adjustment knob 88, a liquid supply tube-detection unit 90, and a liquid supply tube-detection indicator lamp 92 are provided at predetermined positions on an operation panel 82 forming the front surface of the housing 80. The operation panel 82 is formed of a surface that is inclined obliquely upward.

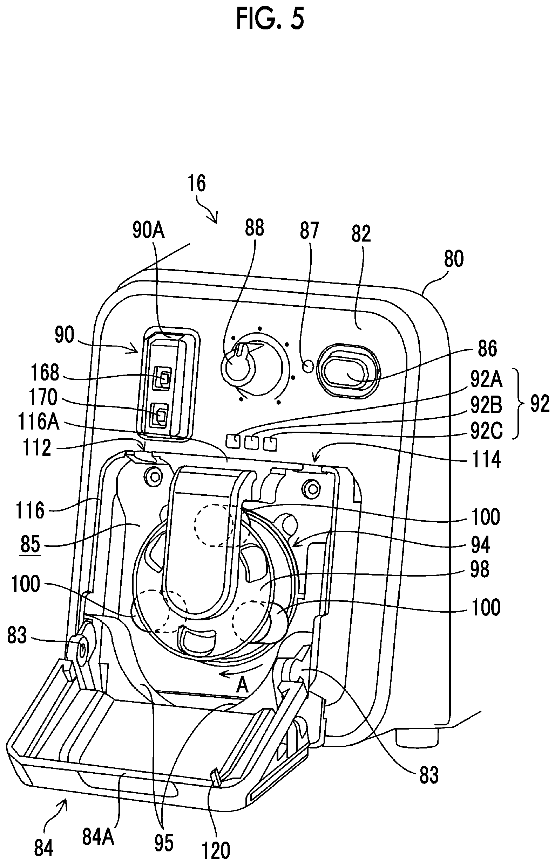

Fig. 5 is an enlarged view of main portions of the liquid supply device body 16 of which the cover 84 is open, and is a diagram showing that a pump 94 having been covered with the cover 84 is exposed to the outside.

As shown in Figs. 4 and 5, the cover 84 is formed in a rectangular shape as a whole and is provided on the liquid supply device body 16 so as to be openable and closable through hinge portions 83 and 83 that are provided on both left and right sides of a lower portion thereof.

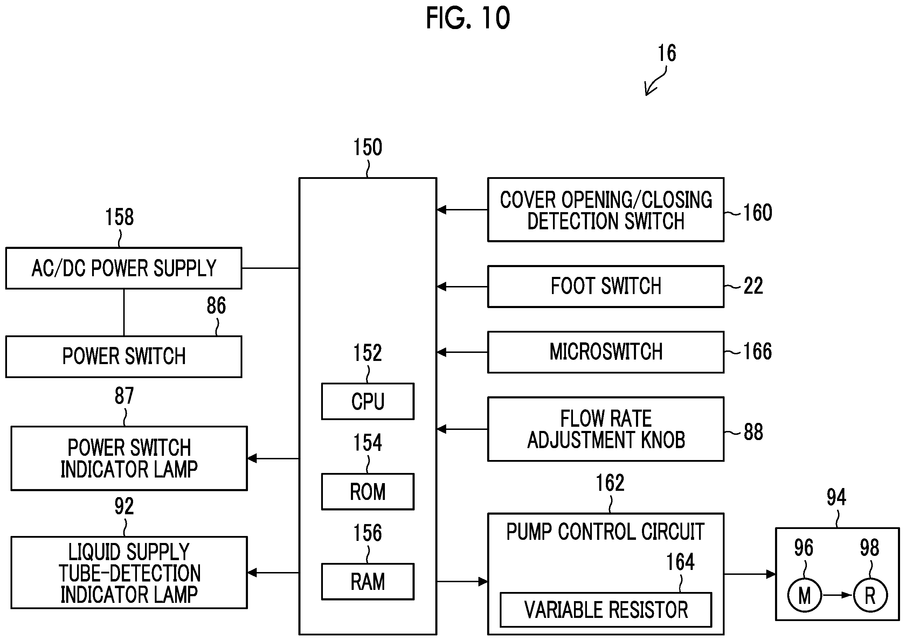

As shown in Fig. 5, the pump 94 is provided in a rotor housing chamber 85 that is exposed to the outside since the cover 84 is open. The pump 94 includes a DC brushless motor (see Fig. 10. Hereinafter abbreviated as a "motor") 96 that is installed in the housing 80, a disc-shaped rotor 98 that is rotated by the motor 96, and three rollers 100, 100, and 100 that are mounted on the outer peripheral portion of the rotor 98 at regular intervals. A liquid supply tube of one tube unit selected from the five types of tube units 300 to 700 is fixed to the pump 94 provided in the liquid supply device body 16 in a state where the liquid supply tube is wound in a U shape on the outer peripheral portion of the rotor 98. The liquid supply tube will be described later.

According to the pump 94 having the above-mentioned configuration, a part of the liquid supply tube is squeezed by the roller 100 and the rotor 98 is rotated in a counterclockwise direction, which is indicated in Fig. 5 by an arrow A, by the motor 96. Accordingly, a position where the liquid supply tube is squeezed is moved in the counterclockwise direction by the rotor 98, so that liquid present in the liquid supply tube is sent to a part to which liquid is to be supplied. In this specification, the water jet pipe 50, the treatment tool-insertion channel 52, and the liquid supply pipe line 74 will be described as an example of the part to which liquid is to be supplied.

The liquid supply device 10 for an endoscope according to the embodiment has the following configuration so that each of the liquid supply tubes of the five types of tube units 300 to 700 is simply attached to and detached from the liquid supply device body 16. The tube unit 300 among the tube units 300 to 700 shown in Fig. 2 will be described below by way of example.

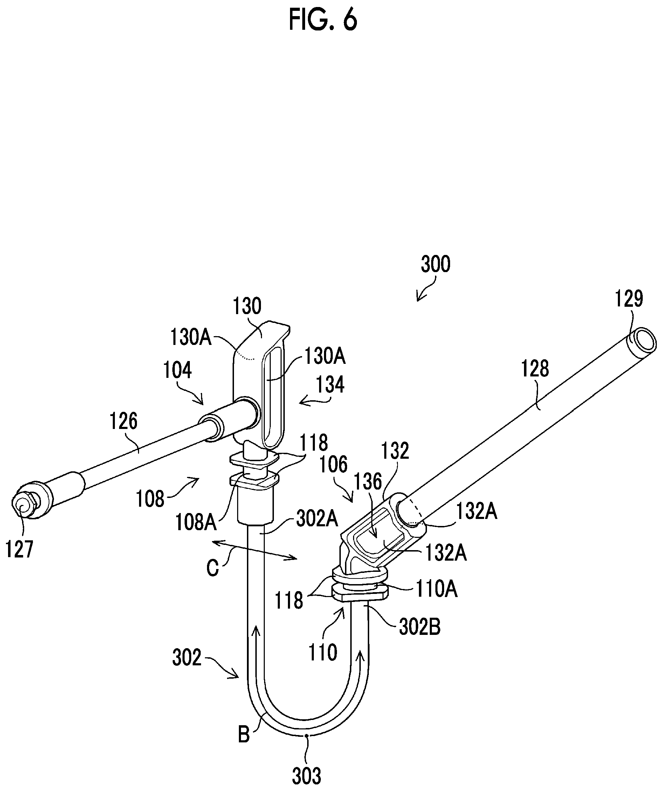

Fig. 6 is a perspective view showing the configuration of the tube unit 300. The same components of the tube units 400 to 700 shown in Fig. 2 as the components of the tube unit 300 shown in Fig. 6 will be denoted by the same reference numerals as those shown in Fig. 6 and the description thereof will be omitted.

The tube unit 300 includes a liquid supply tube 302 that is to be wound on the outer peripheral portion of the rotor 98 (see Fig. 5). The liquid supply tube 302 is an elastic tube made of, for example, silicone rubber, and includes a winding portion 303 that is a portion to be wound on the rotor 98 in a case where the liquid supply tube 302 is to be wound on the rotor 98. Further, a joint 104 is provided at an end portion 302A of the liquid supply tube 302 that is close to a liquid destination side, and a joint 106 is provided at an end portion 302B of the liquid supply tube 302 that is close to a liquid source side. The joint 104 is an example of a first joint and the joint 106 is an example of a second joint.

The joint 104 is provided with a stopper part 108 and the joint 106 is provided with a stopper part 110. Each of these stopper parts 108 and 110 is formed in the shape of a flange protruding in a radial direction of the tube (the direction of an arrow C shown in Fig. 6) orthogonal to an axial direction of the liquid supply tube 302 (the direction of an arrow B shown in Fig. 6).

As shown in Fig. 6, the stopper parts 108 and 110 include shaft portions 108A and 110A extending in the axial direction of the tube (the direction of the arrow B shown in Fig. 6), respectively. Further, the stopper part 108 includes a pair of stopper pieces 118 and 118 that is disposed to be spaced apart from each other in the axial direction of the tube (the direction of the arrow B shown in Fig. 6) with the shaft portion 108A interposed therebetween, and the stopper part 110 includes a pair of stopper pieces 118 and 118 that is disposed to be spaced apart from each other in the axial direction of the tube with the shaft portion 110A interposed therebetween.

As shown in Fig. 5, the liquid supply device body 16 is provided with a restriction portion 112 that is engaged with the stopper part 108 (see Fig. 6) and restricts the position of the stopper part 108 and a restriction portion 114 that is engaged with the stopper part 110 (see Fig. 6) and restricts the position of the stopper part 110. These restriction portions 112 and 114 are provided so as to be spaced apart from each other at an upper side portion 116A of a rectangular housing case 116 that is formed on the operation panel 82 and defines the rotor housing chamber 85.

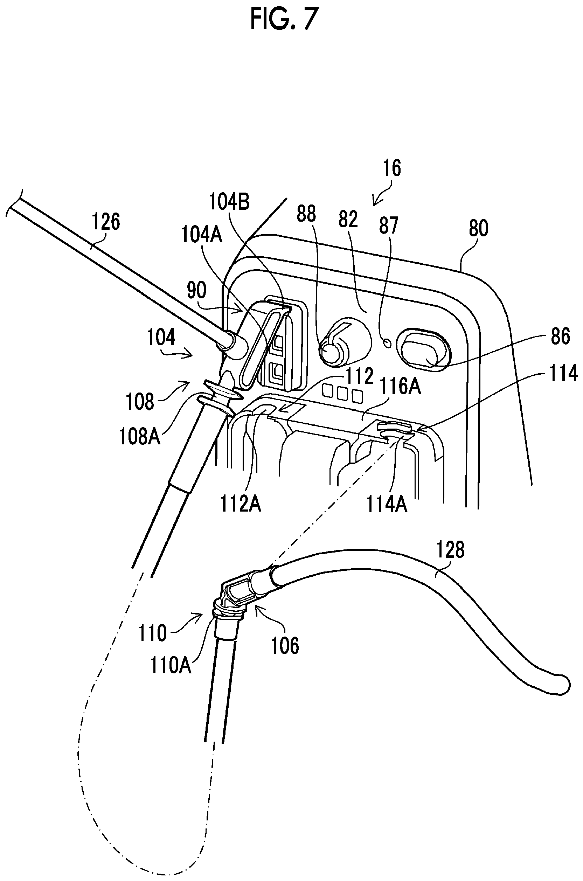

Further, the restriction portions 112 and 114 shown in Fig. 5 include grooves 112A and 114A (see Fig. 7) into which the shaft portions 108A and 110A are to be inserted, respectively. The grooves 112A and 114A are formed by the notching of a part of the upper side portion 116A.

Fig. 7 is a perspective view of which main portions are enlarged and which shows examples of the shapes of the restriction portions 112 and 114.

As shown in Fig. 7, the groove 112A of the restriction portion 112 is formed in a U shape and the groove 114A of the restriction portion 114 is formed in an L shape. Accordingly, the stopper part 108 is engaged with the restriction portion 112 by an operation for pushing the shaft portion 108A into the groove 112A. Further, the stopper part 110 is engaged with the restriction portion 114 by an operation for pushing the shaft portion 110A into the groove 114A in an L shape.

Since the pairs of stopper pieces 118 and 118 are engaged with the upper side portion 116A in this case, the movement of the stopper parts 108 and 110 to both sides in the axial direction of the tube (the direction of the arrow B shown in Fig. 6) is restricted. Since a shift in the position of the liquid supply tube 302 in the axial direction of the tube (the direction of the arrow B shown in Fig. 6) is prevented by this configuration during the rotation of the rotor 98, an operation for stably supplying liquid by the liquid supply device body 16 is achieved.

Further, the diameters of the shaft portions 108A and 110A of the stopper parts 108 and 110 are different from each other, and the widths of the grooves 112A and 114A of the restriction portions 112 and 114 correspond to the diameters of the shaft portions 108A and 110A, respectively. Since the stopper parts 108 and 110 and the restriction portions 112 and 114 are formed as described above, the incorrect attachment of the stopper part 108 to the restriction portion 114 or the incorrect attachment of the stopper part 110 to the restriction portion 112 can be prevented.

According to the liquid supply device 10 for an endoscope of the embodiment, due to this configuration, it is possible to connect the liquid supply tube 302 to the liquid supply device body 16 merely by engaging the stopper part 108 with the restriction portion 112 and engaging the stopper part 110 with the restriction portion 114 in a state where the winding portion 303 is wound on the outer peripheral portion of the rotor 98. Further, it is possible to detach the liquid supply tube 302 from the liquid supply device body 16 merely by detaching the stopper part 108 from the restriction portion 112 and detaching the stopper part 110 from the restriction portion 114.

Furthermore, as shown in Fig. 5, an upper side portion 84A of the cover 84 includes a pressing piece 120 that has the shape of the tip of a sword and protrudes toward the restriction portion 114. In a case where the cover 84 is closed, the pressing piece 120 presses the shaft portion 110A of the stopper part 110 engaged with the groove 114A of the restriction portion 114. Accordingly, the movement of the stopper part 110 in the groove 114A in the radial direction of the tube (the direction of the arrow C shown in Fig. 6) is restricted by the pressing piece 120. Therefore, in a case where the cover 84 is closed, a state where the stopper part 110 is engaged with the restriction portion 114 is maintained. The pressing piece 120 may also be provided on a side corresponding to the restriction portion 112 without being limited to a side corresponding to the restriction portion 114. Accordingly, in a case where the cover 84 is closed, a state where the stopper part 108 is engaged with the restriction portion 112 is maintained as in the case of the restriction portion 114.

Further, each of the joints 104 and 106 shown in Fig. 6 is formed of a coupler that connects tubes. A tube 126 corresponding to the liquid destination side is connected to the joint 104. The tube 126 includes a connecting pipe 127 provided at an end portion thereof opposite to the joint 104, and the connecting pipe 127 is attachably and detachably connected to the forceps port 72 shown in Fig. 2. The tube units 400 to 700 also include the same tubes 126, the tube 126 of the tube unit 400 is attachably and detachably connected to the forceps port 72 through the connecting pipe 127, the tube 126 of each of the tube unit 500 and 600 is attachably and detachably connected to the liquid supply connector 66 through the connecting pipe 127, and the tube 126 of the tube unit 700 is attachably and detachably connected to the proximal end opening 74A of the high-frequency knife 14 through the connecting pipe 127. Here, the tube 126 is an example of a first tube and the connecting pipe 127 is an example of a first connecting part.

Furthermore, a tube 128 corresponding to the liquid source side is connected to the joint 106 shown in Fig. 6. The tube 128 includes a distal end opening part 129 provided at an end portion thereof opposite to the joint 106, and the distal end opening part 129 is inserted into the tank 18 through a connection port 18A of the tank 18 shown in Fig. 2 and is positioned below the liquid level in the tank 18. The tube units 400 to 700 also include the same tubes 128, and the distal end opening part 129 of each of the tube 128 of the tube unit 400, the tubes 128 of the tube units 500 and 600, and the tube 128 of the tube unit 700 is inserted into the tank 18 through the connection port 18A of the tank 18 and is positioned below the liquid level in the tank 18. The tube 128 is an example of a second tube and the distal end opening part 129 is an example of a second connecting part.

As shown in Fig. 6, the joint 104 includes a joint body part 130 having a substantially rectangular parallelepiped shape and the joint 106 includes a joint body part 132 having a substantially rectangular parallelepiped shape. The joint body part 130 includes a knob portion 134 comprising a pair of knob surfaces 130A and 130A opposite to each other, and the joint body part 132 includes a knob portion 136 comprising a pair of knob surfaces 132A and 132A opposite to each other. Since the joints 104 and 106 include the knob portions 134 and 136, an operator can pick up the knob portions 134 and 136 with fingers and easily engage the stopper parts 108 and 110 with the restriction portions 112 and 114.

Here, the tube units 300 to 700 will be described with reference to Fig. 2.

As already described above, the tube units 300 and 400 are connected to the forceps port 72. The tube unit 300 is a reuse type of tube unit that is sterilized and washed and is reused on each examination, and the tube unit 400 is a disposable type of tube unit that is discarded after an examination. Further, the tube units 500 and 600 are connected to the liquid supply connector 66. The tube unit 500 is a reuse type of tube unit that is sterilized and washed and is reused on each examination, and the tube unit 600 is a disposable type of tube unit that is discarded after an examination. Furthermore, the tube unit 700 is connected to the proximal end opening 74A of the high-frequency knife 14, and is a disposable type of tube unit that is discarded after an examination.

Since the inner diameter of the treatment tool-insertion channel 52 shown in Fig. 3 is larger than the inner diameter of each of the water jet pipe 50 and the liquid supply pipe line 74, the amount (mL/min) of liquid supplied by the treatment tool-insertion channel 52 is set to be larger than the amount of liquid supplied by each of the water jet pipe 50 and the liquid supply pipe line 74. Accordingly, the inner diameters of the liquid supply tubes 302 and 402 of the tube units 300 and 400 are larger than the inner diameters of the liquid supply tubes 502, 602, and 702 of the tube units 500, 600, and 700.

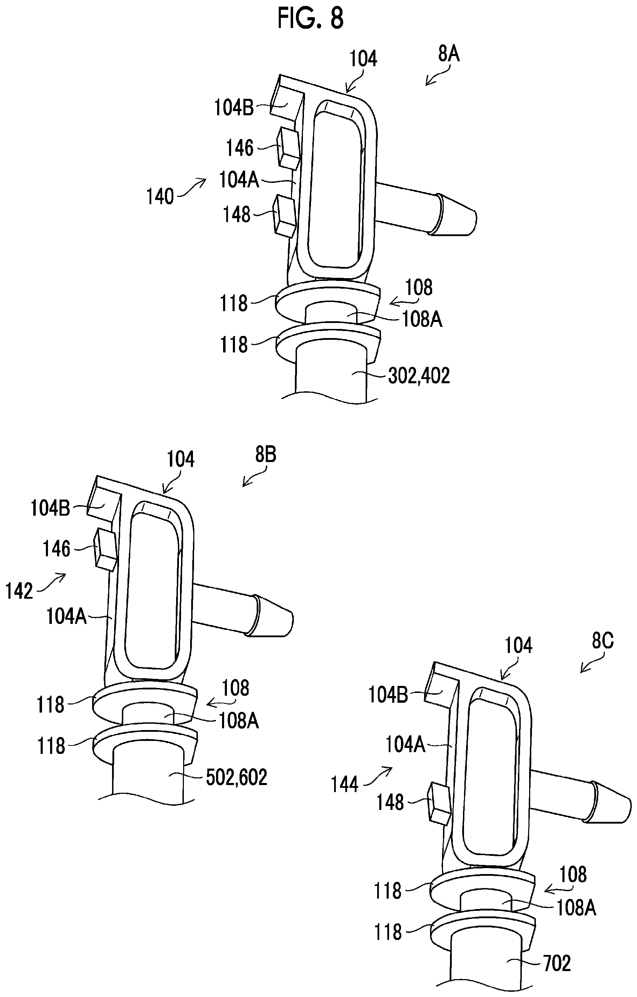

Next, examples of identifiers used to identify the types of the liquid supply tubes 302, 402, 502, 602, and 702 will be described with reference to (A), (B), and (C) of Fig. 8.

(A) of Fig. 8 shows the identifier 140 used to identify each of the liquid supply tubes 302 and 402, (B) of Fig. 8 shows the identifier 142 used to identify each of the liquid supply tubes 502 and 602, and (C) of Fig. 8 shows the identifier 144 used to identify the liquid supply tube 702.

These identifiers 140, 142, and 144 are formed to have different shapes by combinations of two projection portions 146 and 148. Here, the projection portions 146 and 148 are examples of mechanical identification information.

The identifier 140 is the identifier of each of the liquid supply tubes 302 and 402 where two projection portions 146 and 148 are provided on a rear surface 104A of the joint 104 so as to be spaced apart from each other in a vertical direction. Further, the identifier 142 is the identifier of each of the liquid supply tubes 502 and 602 where one projection portion 146 is provided at the upper portion of the rear surface 104A of the joint 104. Furthermore, the identifier 144 is the identifier of the liquid supply tube 702 where one projection portion 148 is provided at the lower portion of the rear surface 104A of the joint 104.

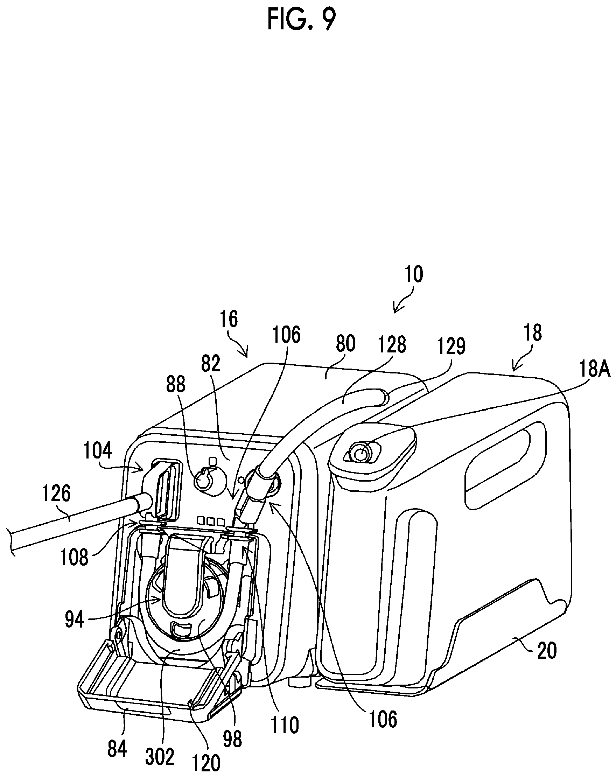

The identifier 140, 142, or 144 is provided at a position that can be recognized by the liquid supply tube-detection unit 90 in a case where the liquid supply tube 302 (402, 502, 602, or 702) is connected to the liquid supply device body 16 as shown in Fig. 9. Then, in a case where the joint 104 is connected to the liquid supply tube-detection unit 90, the identifier 140, 142, or 144 is recognized by an identifier recognition unit of the liquid supply device body 16. The identifier recognition unit will be described later.

A protruding portion 104B is formed at the upper portion of the rear surface 104A of the joint 104 as shown in (A), (B), and (C) of Fig. 8 and a recessed portion 90A (see Fig. 5) is formed at the upper portion of the liquid supply tube-detection unit 90. Accordingly, in a case where the protruding portion 10B is engaged with the recessed portion 90A (see Fig. 7), the joint 104 is easily positioned with respect to the liquid supply tube-detection unit 90.

Fig. 10 is a control block diagram relating to the control of the amount of liquid to be supplied by the liquid supply device body 16.

As shown in Fig. 10, the amount of liquid to be supplied by the liquid supply device body 16 is controlled by a central processing unit (CPU) 152 provided in a control circuit 150. A plurality of circuits, such as a read only memory (ROM) 154 for storing programs and a random access memory (RAM) 156 in which data are temporarily stored, are provided in the control circuit 150.

Data (flow rate range-setting data) used to set the flow rate range of liquid for each type of a liquid supply tube according to a detection signal output from a microswitch 166 to be described later are stored in the ROM 154. Power is supplied to the respective circuits, which are provided in the control circuit 150, from an AC/DC power supply 158.

A cover opening/closing detection switch 160 for detecting the opening/closing of the cover 84, the foot switch 22, the flow rate adjustment knob 88, and a pump control circuit 162 for causing the pump 94 to operate according to the operation position of the flow rate adjustment knob 88 are connected to the control circuit 150. The pump control circuit 162 includes a current switching circuit that switches current flowing in the motor 96, and a variable resistor 164 is used as an example of the current switching circuit. The control circuit 150 causes each circuit to operate in a case where a signal indicating the closing of the cover 84 is output from the cover opening/closing detection switch 160.

The control circuit 150 sets a flow rate, which is sent from the pump 94, in a flow rate range corresponding to the type of the liquid supply tube connected to the microswitch 166 by reading out data from the ROM 154 according to a detection signal output from the microswitch 166 and outputting the data to the pump control circuit 162. Further, the control circuit 150 adjusts a flow rate in the flow rate range according to the operation position of the flow rate adjustment knob 88. Specifically, the flow rate adjustment knob 88 can be operated in an operation range of 0 to 100%. For example, in a case where the flow rate adjustment knob is in an operation position of 50%, the control circuit 150 sets the rotational speed of the rotor 98 so that liquid is supplied at a medium flow rate in the flow rate range. That is, the control circuit 150 sets the amount of liquid to be supplied to the amount of liquid to be supplied (a ratio to the flow rate range) that corresponds to the operation position of the flow rate adjustment knob 88 in the set flow rate range. Accordingly, the amount of liquid to be supplied (a ratio to the flow rate range) corresponding to the operation position of the flow rate adjustment knob 88 is achieved in the flow rate range that is set for each type of a liquid supply tube.

Here, the liquid supply tube-detection unit 90 shown in Fig. 5 includes the microswitch 166 (see Fig. 10) that can mechanically read the projection portions 146 and 148 (see (A), (B), and (C) of Fig. 8).

The microswitch 166 includes two actuators 168 and 170 (see Fig. 5) that are pressed by the projection portions 146 and 148 (see (A), (B), and (C) of Fig. 8) in a case where the joint 104 is connected to the liquid supply tube-detection unit 90 (see Fig. 5) (see Fig. 9), and two terminal parts (not shown) detecting that the actuators 168 and 170 are pressed and outputting a detection signal to the control circuit 150.

Specifically, for example, in a case where the joint 104 of the liquid supply tube 302 is connected to the liquid supply tube-detection unit 90, the actuator 168 is pressed by the projection portion 146 and the actuator 170 is pressed by the projection portion 148. Accordingly, a detection signal indicating that the actuators 168 and 170 are pressed is output to the control circuit 150 from the terminal parts.

The control circuit 150 recognizes the connection of the liquid supply tube 302 to the liquid supply device body 16 on the basis of the detection signal, and changes the operating condition of the pump 94. That is, in a case where the liquid supply tube 302 is connected, the range of the rotational speed of the rotor 98 is set so that the amount of liquid to be supplied is in the flow rate range of, for example, 400 mL/min to 700 mL/min. Then, the amount of liquid to be supplied is set to the amount of liquid to be supplied (a ratio to the flow rate range) that corresponds to the operation position of the flow rate adjustment knob 88 in the set flow rate range. Accordingly, in a case where the foot switch 22 is pressed, the rotor 98 is rotated at a rotational speed corresponding to the amount of liquid to be supplied corresponding to the operation position of the flow rate adjustment knob 88 and liquid corresponding to the rotational speed is sent to the treatment tool-insertion channel 52.

The same applies to a case where the joint 104 of the liquid supply tube 402 is connected to the liquid supply tube-detection unit 90.

Since the actuator 168 is pressed by the projection portion 146 in a case where the joint 104 of the liquid supply tube 502 is connected, a detection signal indicating that the actuator 168 is pressed is output to the control circuit 150 from the terminal parts. Accordingly, the range of the rotational speed of the rotor 98 is set so that the amount of liquid to be supplied, which can be adjusted by the flow rate adjustment knob 88, is in the flow rate range of, for example, 60 mL/min to 190 mL/min. Then, the amount of liquid to be supplied is set to the amount of liquid to be supplied (a ratio to the flow rate range) that corresponds to the operation position of the flow rate adjustment knob 88 in the set flow rate range.

The same applies to a case where the joint 104 of the liquid supply tube 602 is connected to the liquid supply tube-detection unit 90.

Since the actuator 170 is pressed by the projection portion 148 in a case where the joint 104 of the liquid supply tube 702 is connected, a detection signal indicating that the actuator 170 is pressed is output to the control circuit 150 from the terminal parts. Accordingly, the range of the rotational speed of the rotor 98 is set so that the amount of liquid to be supplied, which can be adjusted by the flow rate adjustment knob 88, is in the flow rate range of, for example, 90 mL/min to 190 mL/min. Then, the amount of liquid to be supplied is set to the amount of liquid to be supplied (a ratio to the flow rate range) that corresponds to the operation position of the flow rate adjustment knob 88 in the set flow rate range.

The microswitch 166 and the control circuit 150 are an example of the identifier recognition unit. Further, the microswitch 166 is an example of a mechanical reading unit. Furthermore, the flow rate adjustment knob 88 is an example of an adjustment unit that is used to manually adjust the flow rate of liquid in the flow rate range. Moreover, the control circuit 150 is an example of a flow rate-setting unit. That is, the control circuit 150 sets the flow rate of liquid, which is to be supplied to a part to which liquid is to be supplied, for each type of a liquid supply tube by identifying the type of the liquid supply tube on the basis of a detection signal output from the microswitch 166 (that is, the identifier recognized by the microswitch 166 and the control circuit 150 that are the identifier recognition unit) and changing the operating condition of the pump 94.

Further, the control circuit 150 controls the turn-on of the liquid supply tube-detection indicator lamp 92 on the basis of a detection signal output from the microswitch 166 (that is, an identifier recognized by the microswitch 166 and the control circuit 150 that are the identifier recognition unit). Specifically, in a case where the joint 104 of the liquid supply tube 302 or 402 is connected, the control circuit 150 turns on a lamp 92A (see Fig. 5) of the liquid supply tube-detection indicator lamp 92. Furthermore, in a case where the joint 104 of the liquid supply tube 502 or 602 is connected, the control circuit 150 turns on a lamp 92B (see Fig. 5) of the liquid supply tube-detection indicator lamp 92. Moreover, in a case where the joint 104 of the liquid supply tube 702 is connected, the control circuit 150 turns on a lamp 92C (see Fig. 5) of the liquid supply tube-detection indicator lamp 92. Accordingly, an operator can grasp one of the liquid supply tubes 302 to 702, which is connected to the liquid supply device body 16, merely by checking the turn-on of the lamp 92A, 92B, and 92C.

Next, the action of the liquid supply device 10 for an endoscope according to the embodiment will be described.

A case where the tube unit 300 is selected and used among the tube units 300 to 700 will be described.

First, as shown in Fig. 5, an operator opens the cover 84 of the liquid supply device body 16 to expose the rotor 98 to the outside. Next, the operator winds the winding portion 303 (see Fig. 6) of the liquid supply tube 302 in a U shape on the outer peripheral portion of the rotor 98. Then, as shown in Fig. 7, the operator engages the stopper part 108 with the restriction portion 112 to restrict the position of the stopper part 108 and engages the stopper part 110 with the restriction portion 114 to restrict the position of the stopper part 110.

After that, the operator connects the joint 104 of the tube unit 300 to the liquid supply tube-detection unit 90. Specifically, after engaging the protruding portion 104B of the joint 104 with the recessed portion 90A of the liquid supply tube-detection unit 90 to position the joint 104 on the liquid supply tube-detection unit 90 as shown in Figs. 5 and 7, the operator pushes the joint 104 to the liquid supply tube-detection unit 90 as shown in Fig. 9. Accordingly, the operator can easily and reliably connect the joint 104 to the liquid supply tube-detection unit 90.

Then, the operator closes the cover 84 as shown in Fig. 1. The operator can fix the tube unit 300 to the liquid supply device body 16 with the above-mentioned work.

In this case, the pressing piece 120 shown in Fig. 5 presses the shaft portion 110A of the stopper part 110 to the groove 114A and restricts the movement of the stopper part 110 in the radial direction of the tube (the direction of the arrow C shown in Fig. 6). Accordingly, a shift in the position of the stopper part 110 with respect to the restriction portion 114 in the radial direction of the tube (the direction of the arrow C shown in Fig. 6) can be prevented. Work for connecting the joint 104 shown in Fig. 7 to the liquid supply tube-detection unit 90 may be performed before work for engaging the stopper part 108 with the restriction portion 112.

Further, it is preferable that the positions of the stopper parts 108 and 110 on the liquid supply tube 302 in the axial direction of the tube (the direction of the arrow B shown in Fig. 6) are set to positions where the liquid supply tube 302 is connected to the liquid supply device body 16 without receiving excessive stress (unnecessary tension) in the axial direction of the tube (the direction of the arrow B shown in Fig. 6). In a case where the stopper parts 108 and 110 are provided at these positions, the stopper part 108 is engaged with the restriction portion 112, and the stopper part 110 is engaged with the restriction portion 114, the length and position of the winding portion 303 of the liquid supply tube 302 can be set to an appropriate length and an appropriate position. Accordingly, since work for adjusting the length and position of the winding portion 303 is easily performed and a change in the amount of liquid to be supplied by the pump 94 can be reduced, an operation for stably supplying liquid by the liquid supply device body 16 is achieved.

Next, the operator connects the connecting pipe 127 of the tube 126 shown in Fig. 6 to the forceps port 72, and inserts the distal end opening part 129 of the tube 128 into the tank 18 through the connection port 18A of the tank 18 and positions the distal end opening part 129 below the liquid level in the tank 18. Accordingly, preparation for sending liquid, which is stored in the tank 18, to the treatment tool-insertion channel 52 is completed.

Next, the operator presses the power switch 86 to actuate the liquid supply device body 16. Accordingly, the power switch indicator lamp 87 is turned on. Further, since the cover 84 is closed in this case, the control circuit 150 shown in Fig. 10 recognizes the connection of the liquid supply tube 302 to the liquid supply device body 16 and identifies the liquid supply tube on the basis of a detection signal output from the microswitch 166. Then, the control circuit 150 reads out data, which are required to set the flow rate range of liquid to a flow rate range corresponding to the liquid supply tube 302, from the ROM 154 and outputs the data to the pump control circuit 162. Accordingly, the range of the rotational speed of the rotor 98 is set so that the amount of liquid to be supplied is in the flow rate range of 400 mL/min to 700 mL/min. Further, the control circuit 150 sets the rotational speed of the rotor 98 so that liquid is supplied at a flow rate in the flow rate range according to the operation position of the flow rate adjustment knob 88. In this case, the control circuit 150 turns on the lamp 92A (see Fig. 5) of the liquid supply tube-detection indicator lamp 92 on the basis of the detection signal.

After that, in a case where the operator presses the foot switch 22, the rotor 98 is rotated at a rotational speed corresponding to the amount of liquid to be supplied (a ratio to the flow rate range) that corresponds to the operation position of the flow rate adjustment knob 88. Accordingly, liquid can be sent to the treatment tool-insertion channel 52 in a flow rate range corresponding to the liquid supply tube 302. Then, in a case where the operator wants to finely adjust the amount of liquid to be supplied, the operator operates the flow rate adjustment knob 88. As a result, the amount of liquid to be supplied can be finely adjusted without exceeding the set flow rate range. The same operation applies to a case where the tube unit 400 is fixed to the liquid supply device body 16.

On the other hand, in a case where the tube unit 500 is fixed to the liquid supply device body 16, the control circuit 150 recognizes the connection of the liquid supply tube 502 to the liquid supply device body 16 and identifies the liquid supply tube on the basis of a detection signal output from the microswitch 166. Then, the control circuit 150 reads out data, which are required to set the flow rate range of liquid to a flow rate range corresponding to the liquid supply tube 502, from the ROM 154 and outputs the data to the pump control circuit 162. Accordingly, the range of the rotational speed of the rotor 98 is set so that the amount of liquid to be supplied is in the flow rate range of 60 mL/min to 190 mL/min. Further, the control circuit 150 sets the rotational speed of the rotor 98 so that liquid is supplied at a flow rate in the flow rate range according to the operation position of the flow rate adjustment knob 88. In this case, the control circuit 150 turns on the lamp 92B of the liquid supply tube-detection indicator lamp 92 on the basis of the detection signal.

After that, in a case where the operator presses the foot switch 22, the rotor 98 is rotated at a rotational speed corresponding to the amount of liquid to be supplied (a ratio to the flow rate range) that corresponds to the operation position of the flow rate adjustment knob 88. Accordingly, liquid can be sent to the water jet pipe 50 in a flow rate range corresponding to the liquid supply tube 502. Then, in a case where the operator wants to finely adjust the amount of liquid to be supplied, the operator operates the flow rate adjustment knob 88. As a result, the amount of liquid to be supplied can be finely adjusted without exceeding the set flow rate range. The same operation applies to a case where the tube unit 600 is fixed to the liquid supply device body 16.

Further, in a case where the tube unit 700 is fixed to the liquid supply device body 16, the control circuit 150 recognizes the connection of the liquid supply tube 702 to the liquid supply device body 16 and identifies the liquid supply tube on the basis of a detection signal output from the microswitch 166. Then, the control circuit 150 reads out data, which are required to set the flow rate range of liquid to a flow rate range corresponding to the liquid supply tube 702, from the ROM 154 and outputs the data to the pump control circuit 162. Accordingly, the range of the rotational speed of the rotor 98 is set so that the amount of liquid to be supplied is in the flow rate range of 90 mL/min to 190 mL/min. Further, the control circuit 150 sets the rotational speed of the rotor 98 so that liquid is supplied at a flow rate in the flow rate range according to the operation position of the flow rate adjustment knob 88. In this case, the control circuit 150 turns on the lamp 92C of the liquid supply tube-detection indicator lamp 92 on the basis of the detection signal.

After that, in a case where the operator presses the foot switch 22, the rotor 98 is rotated at a rotational speed corresponding to the amount of liquid to be supplied (a ratio to the flow rate range) that corresponds to the operation position of the flow rate adjustment knob 88. Accordingly, liquid can be sent to the liquid supply pipe line 74 in a flow rate range corresponding to the liquid supply tube 702. Then, in a case where the operator wants to finely adjust the amount of liquid to be supplied, the operator operates the flow rate adjustment knob 88. As a result, the amount of liquid to be supplied can be finely adjusted without exceeding the set flow rate range.

According to the liquid supply device 10 for an endoscope of the embodiment, as described above, the amount of liquid to be supplied can be automatically changed to the amounts of liquid to be supplied that correspond to the changed liquid supply tubes 302 to 702 in a case where the liquid supply tubes 302 and 702 are interchanged with different types of liquid supply tubes 302 to 702.

Accordingly, the liquid supply device 10 for an endoscope according to the embodiment comprises: the microswitch 166 and the control circuit 150 that recognize the identifiers 140 to 144 used to identify the types of the liquid supply tubes 302 to 702; and the control circuit 150 that sets the flow rate of liquid, which is to be supplied to a part to which liquid is to be supplied, for the respective types of the liquid supply tubes 302 to 702 by changing the operating condition of the pump 94 on the basis of the identifiers 140 to 144 recognized by the microswitch 166 and the control circuit 150 (that is, according to the types of the liquid supply tubes 302 to 702). Accordingly, convenience can be improved.

Further, according to the liquid supply device 10 for an endoscope of the embodiment, the flow rate of liquid can be manually adjusted in the flow rate range, which is set for each type of a liquid supply tube by the control circuit 150, by the flow rate adjustment knob 88. Accordingly, the amount of liquid to be supplied can be finely adjusted without exceeding the flow rate range that is set according to the type of a liquid supply tube.

In the embodiment, an aspect where mechanical identifiers are formed to have different shapes by combinations of two projection portions 146 and 148 has been exemplified and the microswitch 166 has been exemplified as the mechanical reading unit. However, other mechanical identifiers and other mechanical reading units may be applied.

For example, mechanical identification information, such as recessed portions or hole portions, may be used as other mechanical identification information, and a mechanical reading unit for reading mechanical identification information, such as the recessed portions or the hole portions, may be used as the mechanical reading unit.

Further, identification information and a reading unit other than the mechanical identification information and the mechanical reading unit may be used as the identification information and the reading unit for reading the identification information. Other examples will be described below.

### First example

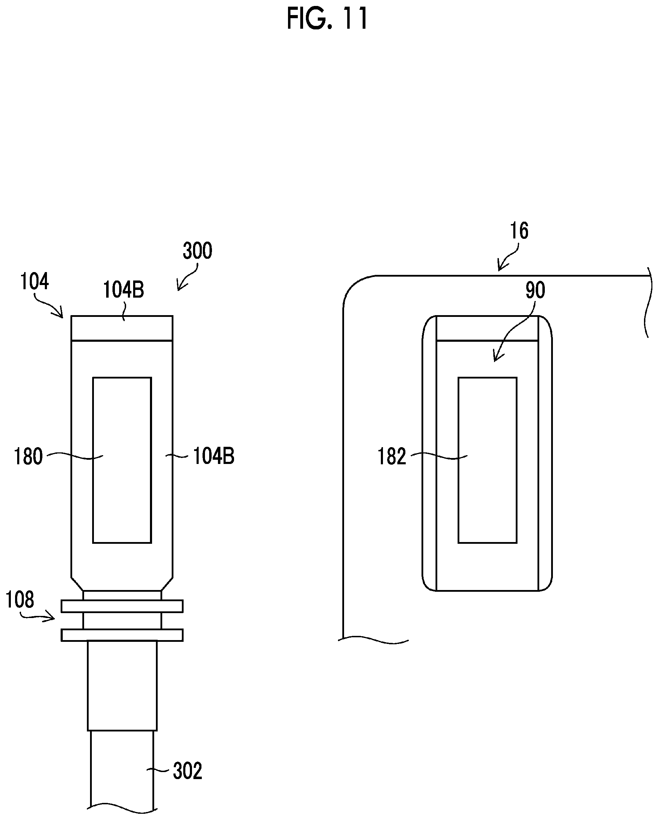

For example, an identifier 180 having magnetic identification information may be provided on a rear surface 104A of a joint 104 as with the joint 104 shown in Fig. 11, and a liquid supply tube-detection unit 90 shown in Fig. 11 may be provided with a reading unit 182 that magnetically reads the magnetic identification information.

The identifier 180 may be mounted on, for example, the liquid supply tube 302 other than the joint 104. According to an aspect where magnetic identification information is used, the degree of freedom in mounting the identifier having magnetic identification information and the reading unit is improved. For example, in a case where the identifier 180 having magnetic identification information is mounted on the liquid supply tube 302, the identifier 180 of the liquid supply tube 302 and the reading unit 182 can be made close to each other as long as the reading unit 182 is mounted in the rotor housing chamber 85. Accordingly, it is preferable that the identifier having magnetic identification information is mounted on the liquid supply tube 302.

A magnetic tape in which magnetic information indicating each of the types of the liquid supply tubes 302 to 702 is magnetically written can be exemplified as the identifier having magnetic identification information. A magnetic information reading unit including a magnetic head, which can read the magnetic information, can be exemplified as the reading unit 182.

### Second example

Alternatively, an identifier 180 having electric identification information may be provided on the rear surface 104A of the joint 104, and the liquid supply tube-detection unit 90 may be provided with a reading unit 182 that electrically reads the electric identification information.

The identifier 180 may be mounted on, for example, the liquid supply tube 302 other than the joint 104. According to an aspect where electric identification information is used, the degree of freedom in mounting the identifier having electric identification information and the reading unit is improved. For example, in a case where the identifier 180 having electric identification information is mounted on the liquid supply tube 302, the identifier 180 of the liquid supply tube 302 and the reading unit 182 can be made close to each other as long as the reading unit 182 is mounted in the rotor housing chamber 85. Accordingly, it is preferable that the identifier having electric identification information is mounted on the liquid supply tube 302.

A radio frequency identifier (RFID) tag including an integrated circuit (IC) chip in which electronic information indicating each of the types of the liquid supply tubes 302 to 702 is stored can be exemplified as the identifier having electric identification information. An electric information reading unit including a reader/writer, which can read the electronic information, can be exemplified as the reading unit 182.

### Third example

Alternatively, an identifier 180 having optical identification information may be provided on the rear surface 104A of the joint 104, and the liquid supply tube-detection unit 90 may be provided with a reading unit 182 that optically reads the optical identification information.

The identifier 180 may be mounted on, for example, the liquid supply tube 302 other than the joint 104. According to an aspect where optical identification information is used, the degree of freedom in mounting the identifier having optical identification information and the reading unit is improved. For example, in a case where the identifier 180 having optical identification information is mounted on the liquid supply tube 302, the identifier 180 of the liquid supply tube 302 and the reading unit 182 can be made close to each other as long as the reading unit 182 is mounted in the rotor housing chamber 85. Accordingly, it is preferable that the identifier having optical identification information is mounted on the liquid supply tube 302. Further, since the rotor housing chamber 85 is shielded from light by the cover 84, the reading unit can read optical identification information without being affected by external light.

A geometric pattern, such as a bar code, a color code, or a dot code, indicating each of the types of the liquid supply tubes 302 to 702 can be exemplified as the identifier having optical identification information; and an optical information reading unit including an image pickup element, such as a laser or a charge coupled device (CCD) capable of reading the geometric pattern, can be exemplified as the reading unit 182.

In the embodiment, the water jet pipe 50, the treatment tool-insertion channel 52, and the liquid supply pipe line 74 have been exemplified as examples of the part to which liquid is to be supplied. However, the part to which liquid is to be supplied is not limited thereto, and may be another liquid supply pipe line provided in the endoscope or a liquid supply pipe line of a treatment tool other than the high-frequency knife, or may be a liquid supply pipe line other than an endoscope and a treatment tool.

The joints 104 and 106 have been provided in the embodiment, but at least one joint may be provided and an identifier may be provided on the joint.

Further, the pump 94 of the embodiment includes arc-shaped wall portions 95 provided below the rotor 98 as shown in Fig. 5, and each of the wall portions 95 is disposed so as to be vertically movable between a retracted position (see Fig. 5) where the wall portion is retracted from the rotor 98 in conjunction with an operation for opening the cover 84 and an advanced position (not shown) where the wall portion pinches the winding portion 303 between the roller 100 and itself in conjunction with an operation for closing the cover 84. Accordingly, in a case where the cover 84 is closed, the winding portion 303 of the liquid supply tube 302 is pinched between the roller 100 and the wall portions 95.

Further, a pump of a type in which each of the liquid supply tubes 302 to 702 is wound on the outer peripheral portion of the rotating rotor 98 has been exemplified as the pump 94 in the embodiment, but any pump that can send liquid through each of the liquid supply tubes 302 to 702 can be applied. For example, in a case where a rotary pump is used as the pump, a plurality of different types of liquid supply tubes are connected to the discharge port of the rotary pump so as to be interchangeable.

The invention has been described above, but it is natural that the invention is not limited to the above-mentioned examples and may have various improvements and modifications without departing from the scope of the invention.

### Explanation of References

10: liquid supply device for endoscope
12: endoscope
14: high-frequency knife
16: liquid supply device body
18: tank
18A: connection port
20: tank tray
22: foot switch
30: endoscope system
32: processor device for endoscope
34: light source device
34A: processor-side connector
35: image processing device
36: display
40: insertion unit
42: soft part
44: bendable part
46: distal end part
46A: distal end face
48: air/water supply channel
50: water jet pipe
52: treatment tool-insertion channel
52A: suction passage
52B: treatment tool-insertion passage
54: operation unit body
56: grip part
58: bending-proof pipe
60: universal cable
62: connector unit
64: electrical connector
66: liquid supply connector
68: air/water supply button
70: suction button
72: forceps port
74: liquid supply pipe line
74A: proximal end opening
76: angle knob
80: housing
82: operation panel
83: hinge portion
84: cover
84A: upper side portion
85: rotor housing chamber
86: power switch
87: power switch indicator lamp
88: flow rate adjustment knob
90: liquid supply tube-detection unit
90A: recessed portion
92: liquid supply tube-detection indicator lamp
94: pump
95: wall portion
96: motor
98: rotor
100: roller
104: joint
104A: rear surface
104B: protruding portion
106: joint
108: stopper part
108A: shaft portion
110: stopper part
110A: shaft portion
112: restriction portion
112A: groove
114: restriction portion
114A: groove
116: housing case
116A: upper side portion
118: stopper piece
120: pressing piece
126: tube
127: connecting pipe
128: tube
129: distal end opening part
130: joint body part
132: joint body part
130A: knob surface
132A: knob surface
134: knob portion
136: knob portion
140: identifier
142: identifier
144: identifier
146: projection portion
148: projection portion
150: control circuit
152: CPU
154: ROM
156: RAM
158: AC/DC power supply
160: cover opening/closing detection switch
162: pump control circuit
164: variable resistor
166: microswitch
168: actuator
170: actuator
180: identifier
182: reading unit
300: tube unit
302: liquid supply tube
302A: end portion
302B: end portion
303: winding portion
400: tube unit
402: liquid supply tube
500: tube unit
502: liquid supply tube
600: tube unit
602: liquid supply tube
700: tube unit
702: liquid supply tube

## Claims

1. A liquid supply tube (302,402,502,602,702) comprising
a first joint (104) which is provided at a first end portion (302A) of the liquid supply tube (302) corresponding to a liquid destination side, and a second joint (106) which is provided at second end portion (302B) of the liquid supply tube (302) corresponding to a liquid source side,
wherein the liquid supply tube (302,402,502,602,702) is configured to be connected to a liquid supply device for an endoscope (10) by the first joint (104) via a liquid supply tube-detection unit (90) of the liquid supply device, the liquid supply device including a liquid supply device body (16), to which a plurality of types of liquid supply tubes are connectable so as to be interchangeable, and sends liquid to a part to which liquid is to be supplied by a pump (94) provided in the liquid supply device body (16),
**characterized in that** the liquid supply tube (302,402,502,602,702) further comprises an identifier (140,142,144) which is used to identify the type of the liquid supply tube (302),
wherein the identifier (140,142,144) is provided at a position that is capable of being recognized by an identifier recognition unit provided in the liquid supply device body (16) in a case where the liquid supply tube (302,402,502,602,702) is connected to the liquid supply device body (16).

2. The liquid supply tube according to claim 1,
wherein the identifier (140,142,144) has mechanical identification information different for each type of the liquid supply tubes, and
the identifier recognition unit is a mechanical reading unit that is capable of mechanically reading the mechanical identification information.

3. The liquid supply tube according to claim 1 or 2,
wherein the identifier (140,142,144) has magnetic identification information different for each type of the liquid supply tubes, and
the identifier recognition unit is a magnetic reading unit that is capable of magnetically reading the magnetic identification information.

4. The liquid supply tube according to claim 1 or 2,
wherein the identifier (140,142,144) has electric identification information different for each type of the liquid supply tubes, and
the identifier recognition unit is an electric reading unit that is capable of electrically reading the electric identification information.

5. The liquid supply tube according to claim 1 or 2,
wherein the identifier (140,142,144) has optical identification information different for each type of the liquid supply tubes, and
the identifier recognition unit is an optical reading unit that is capable of optically reading the optical identification information.

6. The liquid supply tube according to any one of claims 1 to 5,
wherein the part to which liquid is to be supplied is a liquid supply pipe line (74) provided in an endoscope or a treatment tool.

7. A liquid supply device body (16) to which the liquid supply tube (302,402,502,602,702) according to any one of claims 1 to 6 is connectable so as to be interchangeable, the liquid supply device body (16) comprising:
an identifier recognition unit configured to recognize an identifier (140,142,144) provided to the liquid supply tube (302,402,502,602,702) according to any one of claims 1 to 6;
a pump (94) configured to send liquid to a part to which liquid is to be supplied through the liquid supply tube (302,402,502,602,702); and
a flow rate-setting unit configured to set a flow rate of the liquid to be supplied to the part to which liquid is to be supplied, for each type of the liquid supply tubes, by changing an operating condition of the pump (94) on the basis of the identifier (140,142,144) recognized by the identifier recognition unit.

8. The liquid supply device body according to claim 7,
wherein the flow rate-setting unit sets the flow rate of the liquid in a flow rate range different for each type of the liquid supply tubes, and
the liquid supply device (10) comprises an adjustment unit that allows the flow rate of the liquid to be manually adjusted in the flow rate range set by the flow rate-setting unit.

9. The liquid supply device body according to claim 7 or 8,
wherein in a state where the liquid supply tube is wound on a rotor including a plurality of rollers provided on an outer peripheral portion thereof, the pump (94) sends liquid by rotating the rotor and squeezing the liquid supply tube (302,402,502,602,702) with the rollers.

10. The liquid supply device body according to any one of claims 7 to 9,
wherein the identifier (140,142,144) has mechanical identification information different for each type of the liquid supply tubes, and
the identifier recognition unit is a mechanical reading unit that is capable of mechanically reading the mechanical identification information.

11. The liquid supply device body according to any one of claims 7 to 9,
wherein the identifier (140,142,144) has magnetic identification information different for each type of the liquid supply tubes, and
the identifier recognition unit is a magnetic reading unit that is capable of magnetically reading the magnetic identification information.

12. The liquid supply device body according to any one of claims 7 to 9,
wherein the identifier (140,142,144) has electric identification information different for each type of the liquid supply tubes, and
the identifier recognition unit is an electric reading unit that is capable of electrically reading the electric identification information.

13. The liquid supply device body according to any one of claims 7 to 9,
wherein the identifier (140,142,144) has optical identification information different for each type of the liquid supply tubes, and
the identifier recognition unit is an optical reading unit that is capable of optically reading the optical identification information.

14. A liquid supply device for an endoscope (10), in which a plurality of types of liquid supply tubes are connectable to a liquid supply device body (16) so as to be interchangeable, the liquid supply device (10) comprising:
the liquid supply tube (302,402,502,602,702) according to any one of claims 1 to 6; and
the liquid supply device (10) according to any one of claims 7 to 13.

## Patentansprüche

1. Flüssigkeitszufuhr-Rohr (302,402,502,602,702), umfassend:
eine erste Verbindung (104), die an einem ersten Endabschnitt (302A) des Flüssigkeitszufuhr-Rohrs (302), der einer Flüssigkeitszielseite entspricht, vorgesehen ist, und eine zweite Verbindung (106), die an einem zweiten Endabschnitt (302B) des Flüssigkeitszufuhr-Rohrs (302), der einer Flüssigkeitsquellseite entspricht, vorgesehen ist,
wobei das Flüssigkeitszufuhr-Rohr (302,402,502,602,702) so konfiguriert ist, dass es über eine Flüssigkeitszufuhr-Rohr-Detektionseinheit (90) der Flüssigkeitszufuhrvorrichtung mit einer Flüssigkeitszufuhrvorrichtung für ein Endoskop (10) durch die erste Verbindung (104) verbunden ist, wobei die Flüssigkeitszufuhrvorrichtung einen Flüssigkeitszufuhrvorrichtungs-Körper (16) enthält, an den mehrere Typen von Flüssigkeitszufuhr-Rohren so anschließbar sind, dass sie austauschbar sind, und Flüssigkeit zu einem Teil, dem Flüssigkeit zugeführt werden soll, durch eine Pumpe (94), die in dem Flüssigkeitszufuhrvorrichtungs-Körper (16) vorgesehen ist, sendet,
**dadurch gekennzeichnet, dass** das Flüssigkeitszufuhr-Rohr (302,402,502,602,702) ferner einen Identifikator (140,142,144) umfasst, der verwendet wird, um den Typ des Flüssigkeitszufuhr-Rohrs (302) zu identifizieren,
wobei der Identifikator (140,142,144) an einer Position, die in der Lage ist, von einer Identifikatorerkennungseinheit, die an dem Flüssigkeitszufuhrvorrichtungs-Körper (16) vorgesehen ist, in einem Fall, in dem das Flüssigkeitszufuhr-Rohr (302,402,502,602,702) mit dem Flüssigkeitszufuhrvorrichtungs-Körper (16) verbunden ist, erkannt zu werden, vorgesehen ist.

2. Flüssigkeitszufuhr-Rohr nach Anspruch 1,
wobei der Identifikator (140,142,144) mechanische Identifikationsinformationen, die für jeden Typ der Flüssigkeitszufuhr-Rohre unterschiedlich sind, aufweist, und
die Identifikatorerkennungseinheit eine mechanische Leseeinheit ist, die in der Lage ist, die mechanischen Identifikationsinformationen mechanisch zu lesen.

3. Flüssigkeitszufuhr-Rohr nach Anspruch 1 oder 2,
wobei der Identifikator (140,142,144) magnetische Identifikationsinformationen, die für jeden Typ der Flüssigkeitszufuhr-Rohre unterschiedlich sind, aufweist, und
die Identifikatorerkennungseinheit eine magnetische Leseeinheit ist, die in der Lage ist, die magnetischen Identifikationsinformationen magnetisch zu lesen.

4. Flüssigkeitszufuhr-Rohr nach Anspruch 1 oder 2,
wobei der Identifikator (140,142,144) elektrische Identifikationsinformationen, die für jeden Typ der Flüssigkeitszufuhr-Rohre unterschiedlich sind, aufweist, und
die Identifikatorerkennungseinheit eine elektrische Leseeinheit ist, die in der Lage ist, die elektrischen Identifikationsinformationen elektrisch zu lesen.

5. Flüssigkeitszufuhr-Rohr nach Anspruch 1 oder 2,
wobei der Identifikator (140,142,144) optische Identifikationsinformationen, die für jeden Typ der Flüssigkeitszufuhr-Rohre unterschiedlich sind, aufweist, und
die Identifikatorerkennungseinheit eine optische Leseeinheit ist, die in der Lage ist, die optischen Identifikationsinformationen optisch zu lesen.

6. Flüssigkeitszufuhr-Rohr nach einem der Ansprüche 1 bis 5,
wobei der Teil, dem Flüssigkeit zugeführt werden soll, eine Flüssigkeitszufuhr-Rohrleitung (74) ist, die bei einem Endoskop oder einem Behandlungswerkzeug vorgesehen ist.

7. Flüssigkeitszufuhrvorrichtungs-Körper (16), an den das Flüssigkeitszufuhr-Rohr (302,402,502,602,702) nach einem der Ansprüche 1 bis 6 so anschließbar ist, dass es austauschbar ist, wobei der Flüssigkeitszufuhrvorrichtungs-Körper (16) umfasst:
eine Identifikatorerkennungseinheit, die so konfiguriert ist, dass sie einen Identifikator (140,142,144), der an dem Flüssigkeitszufuhr-Rohr (302,402,502,602,702) vorgesehen ist, nach einem der Ansprüche 1 bis 6 erkennt;
eine Pumpe (94), die so konfiguriert ist, dass sie Flüssigkeit zu einem Teil, dem Flüssigkeit durch das Flüssigkeitszufuhr-Rohr (302,402,502,602,702) zugeführt werden soll, sendet; und
eine Strömungsraten-Einstelleinheit, die so konfiguriert ist, dass sie eine Strömungsrate der zu dem Teil, dem Flüssigkeit zugeführt werden soll, für jeden Typ der Flüssigkeitszufuhr-Rohre einstellt, indem sie eine Betriebsbedingung der Pumpe (94) auf der Grundlage des Identifikators (140, 142, 144), der von der Identifikatorerkennungseinheit erkannt wird, ändert.

8. Flüssigkeitszufuhrvorrichtungs-Körper nach Anspruch 7,
wobei die Strömungsraten-Einstelleinheit die Strömungsrate der Flüssigkeit in einem Strömungsratenbereich, der für jeden Typ der Flüssigkeitszufuhr-Rohre unterschiedlich ist, einstellt, und
die Flüssigkeitszufuhrvorrichtung (10) eine Anpassungseinheit umfasst, die es ermöglicht, dass die Strömungsrate der Flüssigkeit in dem von der Strömungsraten-Einstelleinheit eingestellten Strömungsratenbereich manuell angepasst werden kann.

9. Flüssigkeitszufuhrvorrichtungs-Körper nach Anspruch 7 oder 8,
wobei in einem Zustand, in dem das Flüssigkeitszufuhr-Rohr auf einem Rotor, der mehrere Rollen, die an einem Außenumfangsabschnitt davon vorgesehen sind, enthält, gewickelt ist, die Pumpe (94) Flüssigkeit sendet, indem sie den Rotor dreht und das Flüssigkeitszufuhr-Rohr (302,402,502,602,702) mit den Rollen quetscht.

10. Flüssigkeitszufuhrvorrichtungs-Körper nach einem der Ansprüche 7 bis 9,
wobei der Identifikator (140,142,144) mechanische Identifikationsinformationen, die für jeden Typ der Flüssigkeitszufuhr-Rohre unterschiedlich sind, aufweist, und
die Identifikatorerkennungseinheit eine mechanische Leseeinheit ist, die in der Lage ist, die mechanischen Identifikationsinformationen mechanisch zu lesen.

11. Flüssigkeitszufuhrvorrichtungs-Körper nach einem der Ansprüche 7 bis 9,
wobei der Identifikator (140,142,144) magnetische Identifikationsinformationen, die für jeden Typ der Flüssigkeitszufuhr-Rohre unterschiedlich sind, aufweist, und
die Identifikatorerkennungseinheit eine magnetische Leseeinheit ist, die in der Lage ist, die magnetischen Identifikationsinformationen magnetisch zu lesen.

12. Flüssigkeitszufuhrvorrichtungs-Körper nach einem der Ansprüche 7 bis 9,
wobei der Identifikator (140,142,144) elektrische Identifikationsinformationen, die für jeden Typ der Flüssigkeitszufuhr-Rohre unterschiedlich sind, aufweist, und
die Identifikatorerkennungseinheit eine elektrische Leseeinheit ist, die in der Lage ist, die elektrischen Identifikationsinformationen elektrisch zu lesen.

13. Flüssigkeitszufuhrvorrichtungs-Körper nach einem der Ansprüche 7 bis 9,
wobei der Identifikator (140,142,144) optische Identifikationsinformationen, die für jeden Typ der Flüssigkeitszufuhr-Rohre unterschiedlich sind, aufweist, und
die Identifikatorerkennungseinheit eine optische Leseeinheit ist, die in der Lage ist, die optischen Identifikationsinformationen optisch zu lesen.

14. Flüssigkeitszufuhrvorrichtung für ein Endoskop (10), bei dem mehrere Typen von Flüssigkeitszufuhr-Rohren mit einem Flüssigkeitszufuhrvorrichtungs-Körper (16) so verbindbar sind, dass sie austauschbar sind, wobei die Flüssigkeitszufuhrvorrichtung (10) umfasst:
das Flüssigkeitszufuhr-Rohr (302,402,502,602,702) nach einem der Ansprüche 1 bis 6; und
die Flüssigkeitszufuhrvorrichtung (10) nach einem der Ansprüche 7 bis 13.

## Revendications

1. Tube d'alimentation de liquide (302, 402, 502, 602, 702) comprenant
un premier joint (104) qui est prévu à une première partie d'extrémité (302A) du tube d'alimentation de liquide (302) correspondant à un côté de destination de liquide, et un deuxième joint (106) qui est prévu à une deuxième partie d'extrémité (302B) du tube d'alimentation de liquide (302) correspondant à un côté de source de liquide,
dans lequel le tube d'alimentation de liquide (302, 402, 502, 602, 702) est configuré pour être connecté à un dispositif d'alimentation de liquide pour un endoscope (10) par le première joint (104) via une unité de détection de tube d'alimentation de liquide (90) du dispositif d'alimentation de liquide, le dispositif d'alimentation de liquide incluant un corps de dispositif d'alimentation de liquide (16), auquel une pluralité de types de tubes d'alimentation de liquide sont connectables de manière à être interchangeables, et envoie du liquide à une partie à laquelle du liquide doit être fourni par une pompe (94) prévue dans le corps de dispositif d'alimentation de liquide (16),
**caractérisé en ce que** le tube d'alimentation de liquide (302, 402, 502, 602, 702) comprend en outre un identifiant (140, 142, 144) qui est utilisé pour identifier le type du tube d'alimentation de liquide (302),
dans lequel l'identifiant (140, 142, 144) est prévu à une position qui est capable d'être reconnue par une unité de reconnaissance d'identifiant prévue dans le corps de dispositif d'alimentation de liquide (16) dans un cas où le tube d'alimentation de liquide (302, 402, 502, 602, 702) est connecté au corps de dispositif d'alimentation de liquide (16).

2. Tube d'alimentation de liquide selon la revendication 1,
dans lequel l'identifiant (140, 142, 144) a des informations d'identification mécanique différentes pour chaque type des tubes d'alimentation de liquide, et
l'unité de reconnaissance d'identifiant est une unité de lecture mécanique qui est capable de lire mécaniquement les informations d'identification mécanique.

3. Tube d'alimentation de liquide selon la revendication 1 ou la revendication 2,
dans lequel l'identifiant (140, 142, 144) a des informations d'identification magnétique différentes pour chaque type des tubes d'alimentation de liquide, et
l'unité de reconnaissance d'identifiant est une unité de lecture magnétique qui est capable de lire magnétiquement les informations d'identification magnétique.

4. Tube d'alimentation de liquide selon la revendication 1 ou la revendication 2,
dans lequel l'identifiant (140, 142, 144) a des informations d'identification électrique différentes pour chaque type des tubes d'alimentation de liquide, et
l'unité de reconnaissance d'identifiant est une unité de lecture électrique qui est capable de lire électriquement les informations d'identification électrique.

5. Tube d'alimentation de liquide selon la revendication 1 ou la revendication 2,
dans lequel l'identifiant (140, 142, 144) a des informations d'identification optique différentes pour chaque type des tubes d'alimentation de liquide, et
l'unité de reconnaissance d'identifiant est une unité de lecture optique qui est capable de lire optiquement les informations d'identification optique.

6. Tube d'alimentation de liquide selon l'une quelconque des revendications 1 à 5,
dans lequel la partie à laquelle du liquide doit être fourni est une conduite d'alimentation de liquide (74) prévue dans un endoscope ou dans un outil de traitement.

7. Corps de dispositif d'alimentation de liquide (16) auquel le tube d'alimentation de liquide (302, 402, 502, 602, 702) selon l'une quelconque des revendications 1 à 6 est connectable de manière à être interchangeable, le corps de dispositif d'alimentation de liquide (16) comprenant :
une unité de reconnaissance d'identifiant configurée pour reconnaître un identifiant (140, 142, 144) prévu au tube d'alimentation de liquide (302, 402, 502, 602, 702) selon l'une quelconque des revendications 1 à 6 ;
une pompe (94) configurée pour envoyer du liquide à une partie à laquelle du liquide doit être fourni à travers le tube d'alimentation de liquide (302, 402, 502, 602, 702) ; et
une unité de réglage de débit configurée pour régler un débit du liquide à fournir à la partie à laquelle du liquide doit être fourni, pour chaque type des tubes d'alimentation de liquide, en modifiant une condition de fonctionnement de la pompe (94) sur la base de l'identifiant (140, 142, 144) reconnu par l'unité de reconnaissance d'identifiant.

8. Corps de dispositif d'alimentation de liquide selon la revendication 7,
dans lequel l'unité de réglage de débit règle le débit du liquide dans une plage de débit différente pour chaque type des tubes d'alimentation de liquide, et
le dispositif d'alimentation de liquide (10) comprend une unité d'ajustement qui permet de régler manuellement le débit du liquide dans la plage de débit réglé par l'unité de réglage de débit.

9. Corps de dispositif d'alimentation de liquide selon la revendication 7 ou la revendication 8,
dans lequel dans un état où le tube d'alimentation de liquide est enroulé sur un rotor incluant une pluralité de rouleaux prévus sur une partie périphérique extérieure de celui-ci, la pompe (94) envoie du liquide en faisant tourner le rotor et en comprimant le tube d'alimentation de liquide (302, 402, 502, 602, 702) avec les rouleaux.

10. Corps de dispositif d'alimentation de liquide selon l'une quelconque des revendications 7 à 9,
dans lequel l'identifiant (140, 142, 144) a des informations d'identification mécanique différentes pour chaque type des tubes d'alimentation de liquide, et
l'unité de reconnaissance d'identifiant est une unité de lecture mécanique qui est capable de lire mécaniquement les informations d'identification mécanique.

11. Corps de dispositif d'alimentation de liquide selon l'une quelconque des revendications 7 à 9,
dans lequel l'identifiant (140, 142, 144) a des informations d'identification magnétique différentes pour chaque type des tubes d'alimentation de liquide, et
l'unité de reconnaissance d'identifiant est une unité de lecture magnétique qui est capable de lire magnétiquement les informations d'identification magnétique.

12. Corps de dispositif d'alimentation de liquide selon l'une quelconque des revendications 7 à 9,
dans lequel l'identifiant (140, 142, 144) a des informations d'identification électrique différentes pour chaque type des tubes d'alimentation de liquide, et
l'unité de reconnaissance d'identifiant est une unité de lecture électrique qui est capable de lire électriquement les informations d'identification électrique.

13. Corps de dispositif d'alimentation de liquide selon l'une quelconque des revendications 7 à 9,
dans lequel l'identifiant (140, 142, 144) a des informations d'identification optique différentes pour chaque type des tubes d'alimentation de liquide, et
l'unité de reconnaissance d'identifiant est une unité de lecture optique qui est capable de lire optiquement les informations d'identification optique.

14. Dispositif d'alimentation de liquide pour un endoscope (10), dans lequel une pluralité de types de tubes d'alimentation de liquide sont connectables à un corps de dispositif d'alimentation de liquide (16) de manière à être interchangeables, le dispositif d'alimentation de liquide (10) comprenant :
le tube d'alimentation de liquide (302, 402, 502, 602, 702) selon l'une quelconque des revendications 1 à 6 ; et
le dispositif d'alimentation de liquide (10) selon l'une quelconque des revendications 7 à 13.
